# FASCICULE DE BREVET EUROPEEN

(11) **EP 2 448 682 B1**
(45) Date de publication et mention de la délivrance du brevet: **25.06.2014**
(21) Numéro de dépôt: 10734938.3
(22) Date de dépôt: 30.06.2010
(51) Int. Cl.: B05B 7/00, B05B 7/04, B05B 7/08, B05B 7/24, F23D 11/10, F23D 11/38, A61M 11/00, A61M 11/02, A61M 11/06

(54) **BUSE DE PULVERISATION DIPHASIQUE ET APPAREIL DE NEBULISATION LA COMPORTANT**
ZWEIPHASEN-SPRÜHDÜSE UND EIN DIESE BEINHALTENDER SPRÜHNEBLER
TWO-PHASE SPRAYING NOZZLE AND VAPOURISING DEVICE COMPRISING THE SAME

(30) Priorité: 30.06.2009 FR 0903196
(43) Date de publication de la demande: 09.05.2012
(73) Titulaire: Benalikhoudja, Karim, 64170 Serres Sainte Marie (FR)
(72) Inventeur: Benalikhoudja, Karim, 64170 Serres Sainte Marie (FR)
(74) Mandataire: Cornuejols, Georges
(86) Numéro de dépôt international: PCT/EP2010/059282
(87) Numéro de publication internationale: WO 2011/000868

(56) Documents cités:
- EP-A- 0 101 109
- DE-A1- 2 627 880
- DE-A1-102006 001 319
- FR-A- 972 990
- US-A- 5 337 962
- US-A1- 2008 283 049
- US-A1- 2009 050 141
- "HYBRID AERATED LIQUID ATOMIZER" RESEARCH DISCLOSURE, MASON PUBLICATIONS, HAMPSHIRE, GB, no. 354, 1 octobre 1993 (1993-10-01), page 686, XP000412838 ISSN: 0374-4353

## Description

### Domaine technique

La présente invention est du domaine des matériels utilisés pour diffuser dans l'atmosphère un aérosol de fines particules liquides et concerne plus particulièrement une buse diphasique de pulvérisation d'un liquide et un appareil de nébulisation équipé d'une buse conforme à l'invention.

Il y a lieu de rappeler que par nébulisation il faut entendre une opération visant à la formation d'un aérosol dont la taille des particules liquides est inférieure à 5 µm.

### Etat de la technique antérieure.

Typiquement les buses de pulvérisation diphasiques comprennent un corps dans lequel sont formés un premier perçage d'amenée d'un gaz porteur, un second perçage d'amenée du liquide à pulvériser, en relation de communication avec un réservoir de liquide et une chambre de fractionnement en relation de communication avec les deux perçages dans laquelle le liquide est divisé en fines gouttelettes par le gaz porteur. Cette chambre possède un orifice de sortie d'un jet de pulvérisation formé de particules liquides en suspension dans un courant de gaz porteur. Généralement, ces buses utilisent l'effet Venturi afin que sous l'effet de la vitesse d'écoulement du gaz propulseur, le liquide à pulvériser soit aspiré dans la chambre de fractionnement. Ces buses ne sont généralement pas conçues pour former des particules de tailles calibrées, au contraire, elles disposent d'une géométrie et d'un ajutage autorisant une grande variabilité de la taille de ces dernières. Ainsi la taille de ces particules peut être comprise entre 0,2 µm et 10 µm.

En vue de la formation d'une nébulisation, cette buse est fonctionnellement associée à une chambre de détente et de piégeage dont la fonction essentielle est d'assurer un tri dimensionnel des particules liquide afin que soit délivré en sortie de chambre, un aérosol composé d'une majorité de particules de tailles inférieures à un calibre déterminé. Les particules de tailles supérieures à ce calibre sont capturées dans la chambre par divers moyens et retournent sous forme d'un écoulement résiduel vers un réservoir de liquide fonctionnellement associé à la buse de pulvérisation. L'ensemble fonctionnel composé d'une buse et d'une chambre de nébulisation est communément dénommé « Tête de nébulisation ».

Ces têtes de nébulisation pemettent dans le plus souvent des cas, en fonction de la nature du liquide, de diffuser dans une atmosphère ambiante une substance odorante, par exemple un parfum, un insecticide, ou bien encore un désinfectant pour la décontamination bactériologique de l'air et des surfaces des locaux. Elles peuvent aussi être utilisées pour nébuliser des substances curatives à visée thérapeutique, par exemple pour le traitement des voies respiratoires des malades en aérosolthérapie, ou bien à visée vétérinaire par exemple pour la vaccination des animaux d'un élevage.

Elles peuvent aussi être utilisées pour la formation d'un mélange, sous forme de brouillard, d'un combustible liquide avec un gaz comburant en vue de l'alimentation de machines thermiques.

On connaît de la demande de brevet FR 2 700 482 un micro-diffuseur pour brouillard de particules liquide doté d'une buse de pulvérisation comprenant une chambre ouverte de pulvérisation dans laquelle sont simultanément introduits au travers d'ajutages appropriés un flux de gaz propulseur sous pression et un flux de liquide à fractionner. En raison de la position des ajutages l'un par rapport à l'autre et de la vitesse d'écoulement du gaz propulseur, une dépression est créée en aval de l'ajutage de sortie de liquide. Sous l'effet de cette dépression, le liquide, par effet Venturi, est aspiré vers la chambre de fractionnement et y est divisé en fines particules par le gaz propulseur sous pression.

La chambre de fractionnement est de forme tronc-conique et les deux ajutages sont ménagés à la base de cette dernière, pour l'un, celui du gaz propulseur, de manière axiale et pour l'autre, celui du liquide, de manière radiale.

La chambre de nébulisation que comporte le micro-diffuseur selon cette demande de brevet, présente une succession de chicanes aptes à piéger les grosses particules.

On connaît aussi de la demande de brevet GB 552 689, un dispositif de pulvérisation ou de vaporisation mettant en oeuvre une buse de pulvérisation comprenant un corps de buse dans lequel est formé un premier perçage axial débouchant dans un épanouissement supérieur et des seconds perçages latéraux au perçage axial, connectés à ce dernier par des conduits radiaux, coplanaires, chaque perçage latéral et le canal radial associé formant un conduit d'amené de liquide en relation de communication avec un réservoir de liquide. Le perçage axial est prévu pour recevoir un écoulement d'air sous pression sous l'effet de la vitesse de déplacement duquel une dépression est formée dans les canaux radiaux et du liquide se trouve aspiré et ensuite, fractionné et entraîné par le flux d'air sous pression vers l'épanouissement supérieur. Le jet de particules liquides ainsi formé est introduit dans une chambre de nébulisation et vient frapper dans cette dernière la paroi d'un tube de sortie. Cette disposition assure le piégeage des grosses particules liquides.

On connaît également de la demande de brevet DE 10 2006 001319 une buse de pulvérisation dont le liquide est introduit sous pression au travers d'un perçage axial. Le gaz est également introduit sous pression par passage au travers de plusieurs perçages radiaux.

La buse fait donc appel pour son fonctionnement à deux sources de pression.

### Exposé de l'invention

### Problème technique.

Les buses diphasiques dédiées à la nébulisation, connues de l'état de la technique, présentent un certains nombre d'inconvénients. Notamment il a été observé pour la plupart d'entre elles une instabilité du jet formé et un mauvais rendement.

L'instabilité du jet résulte essentiellement soit d'une alimentation déséquilibrée en liquide et/ou soit d'un débit irrégulier en alimentation. Pour la buse selon la demande de brevet FR 2 700 482, la position radiale de l'arrivée de liquide entraîne une asymétrie du jet formé avec une distribution non uniforme des particules alors que ce dernier devrait être parfaitement conique. Pour la buse selon le GB 552 689, l'obturation partielle ou totale de l'un des perçages d'amenée de liquide peut se traduire par un déséquilibre du jet et par un fractionnement irrégulier ou erratique du liquide.

Le mauvais rendement de la buse résulte essentiellement d'une trop faible proportion, seulement de l'ordre de 15%, de particules de tailles appropriées à savoir inférieure à 5 µm.

Pour compenser les faibles rendements, il est connu d'augmenter le débit du courant porteur au travers de la buse et pour cette raison, d'utiliser des compresseurs d'air surdimensionnés par rapport à l'application souhaitée. Ceci se traduit par une consommation énergétique accrue.

Un autre inconvénient des buses diphasiques de l'art antérieur tient au fait qu'il est difficile de maîtriser la hauteur d'aspiration ceci en raison de leur configuration qui ne permet l'obtention d'une dépression constante et adéquate.

Encore un autre inconvénient des buses actuelles tient au fait qu'elles ne sont pas reproductibles en raison de leurs profils géométriques extrêmement difficiles à réaliser et à contrôler. Ce défaut se traduit, d'une buse à l'autre, par des dispersions de résultats en termes de qualité de pulvérisation. Pour pallier cet inconvénient, la position du point d'ajutage entre l'arrivé d'air et de liquide peut être ajustable mais cette opération de mise au point est difficilement maîtrisable compte tenu de l'étroitesse des plages dimensionnelles concernées. En outre il arrive que les réglages initiaux s'altèrent dans le temps sous l'effet notamment de l'usure et des vibrations que subit la buse principalement lors de son fonctionnement. Il s'ensuit des modifications de comportement préjudiciables à un bon rendement.

Enfin, les buses connues ne peuvent pas produire de jet de particules sous une plage de débit étendue.

### Solution technique.

La présente invention a pour objet de résoudre les inconvénients précédemment cités en mettant en oeuvre une buse apte à produire un jet parfaitement conique, homogène et parfaitement stable.

Un autre but de la présente invention est la mise en oeuvre d'une buse présentant un bon rendement.

Un autre but de la présente invention est une conception de buse avec laquelle est obtenue une reproductibilité des résultats d'une buse à l'autre.

Un autre but de la présente invention est la mise en oeuvre d'une buse pour laquelle aucun réglage n'est requis.

À cet effet, la buse de pulvérisation selon l'invention, formée d'un corps de buse comportant un premier perçage d'amenée de gaz sous pression présentant un orifice d'entrée et un orifice de sortie, au moins un second perçage d'amenée d'un liquide à fractionner présentant un orifice d'entrée et un orifice de sortie, une zone de fractionnement du liquide, axialement alignée avec le premier perçage selon un axe géométrique AA', ladite zone de fractionnement étant en relation de communication avec les premier et second perçages se caractérise essentiellement en ce qu'elle comporte plusieurs perçages d'amenée du liquide, que chacun de ces perçages est en relation de communication avec le premier perçage et avec la zone de fractionnement au travers d'une même chambre de dépression formée entre ledit premier perçage et la zone de fractionnement, ladite chambre de dépression étant traversée par le flux de gaz propulseur et le diamètre de la zone de fractionnement étant plus important que le diamètre du premier perçage au niveau de son débouché dans la chambre de dépression.

Sous l'effet de la vitesse du gaz propulseur, par effet Venturi, une dépression est créée dans la chambre de dépression et le liquide à pulvériser est aspiré vers cette dernière. En raison de la présence de cette chambre, qui constitue une réserve tampon de liquide, est assurée la régularité du débit du jet de pulvérisation en sortie de buse.

Une telle buse présente l'avantage d'une fabrication particulièrement simple et facilement contrôlable et totalement reproductible sans aucun réglage.

En outre cette buse peut être réalisée dans les technologies d'injection plastique afin d'optimiser les coûts de fabrication et de pouvoir être intégrée dans des cartouches de nébulisation à usage unique.

En raison de la présence de la chambre de dépression, la dépression obtenue est relativement importante compte tenu de la vitesse du jet de gaz propulseur de sorte que pour la mise en pression de ce gaz pourra être utilisé un compresseur de moindre puissance.

À titre d'exemple purement indicatif avec un micro compresseur fonctionnant sous une tension de 12 Vcc et apte à produire un jet de gaz porteur sous une pression de 250 mbars sera aspiré un liquide sur une hauteur de 1 mètre. Pour des applications de nébulisation, l'aspiration du liquide avec un micro compresseur fonctionnant sous 12 Vcc est de 20 mbars sur une hauteur de dépression de 160 mm.

Selon une autre caractéristique de l'invention, l'ajutage du premier perçage au niveau du débouché de ce dernier dans la chambre de dépression est axial à ladite chambre et les ajutages des seconds perçages au niveau des débouchés de ces derniers dans ladite chambre sont disposés symétriquement par rapport à l'axe AA'.

Selon une autre caractéristique de l'invention, le débit de liquide en atomisation ou en nébulisation est réglable en augmentant le volume de la chambre de dépression ou en multipliant le nombre d'orifices d'arrivée de liquide dans la chambre ce choix étant déterminé par l'installation de la buse sur son support. Une telle disposition présente l'avantage d'une grande flexibilité dans sa mise en place.

Cette disposition de répartition équilibrée des ajutages permet l'alimentation homogène en liquide de la zone de fractionnement et par voie de conséquence la formation d'un jet parfaitement conique et homogène.

Selon une autre caractéristique de l'invention, le volume de la chambre de dépression est ajustable. On peut ajuster ainsi le volume du tampon de liquide mais aussi pour un débit de gaz propulseur donné, la valeur de la dépression régnant dans la cette chambre et par voie de conséquence le débit du liquide aspiré.

Selon une autre caractéristique de l'invention, l'ajustement du volume de la chambre de dépression est opéré par variation de la hauteur de cette dernière.

Selon une autre caractéristique de l'invention, le corps de buse est formé de deux parties jointes de manière étanche l'une à l'autre entre lesquelles est formée la chambre de dépression, la variation de hauteur de cette dernière, selon une autre caractéristique de l'invention, étant opérée par déplacement axial des deux parties l'une par rapport à l'autre.

Selon une autre caractéristique de l'invention, la zone de fractionnement est formée par un perçage débouchant dans la chambre de dépression et ce dans l'axe du premier perçage.

Selon une autre caractéristique de l'invention, le perçage constituant la zone de fractionnement présente au niveau de son débouché dans la chambre de dépression et en regard du premier perçage, un évasement conique. Cette disposition a pour effet d'augmenter l'efficacité du fractionnement du liquide et de la mise en dépression de la chambre de dépression.

Selon une autre caractéristique de l'invention, le corps de buse, radialement au jet de pulvérisation peut recevoir un tube qui présente au moins deux prises d'air débouchant en regard de la sortie d'atomisation du liquide, les dites prises d'air étant régulièrement réparties autour de l'axe de symétrie.

Cette disposition, par effet Venturi, permet une introduction d'air dans le jet produit afin d'en augmenter le débit d'air en sortie de buse.

La présente invention est également relative à un appareil comportant au moins une buse selon l'invention.

Selon une autre caractéristique de l'invention, l'appareil comprend un corps creux d'appareil pourvu d'un embout d'emmanchement sur le col d'un réservoir de liquide, d'une embase transversale disposée au-dessus de l'embout d'emmanchement dans laquelle est formé au moins un logement apte à recevoir en emboîtement de forme une buse de pulvérisation.

### Description sommaire des figures et des dessins.

D'autres avantages, buts et caractéristiques de l'invention apparaîtront à la lecture de la description d'une forme préférée de réalisation, donnée à titre d'exemple non limitatif en se référant aux dessins annexés en lesquels :
- la figure 1 est une vue en coupe longitudinale d'une buse selon une première forme de réalisation,
- la figure 1a est une vue en perspective d'une buse selon la figure 1,
- la figure 2 est une vue en coupe longitudinale d'une buse selon une seconde forme de réalisation,
- la figure 2a est une vue en perspective d'une buse selon la figure 2,
- la figure 3 est une vue en coupe longitudinale d'une buse selon une troisième forme de réalisation,
- la figure 3a est une vue en perspective d'une buse selon la figure 3,
- la figure 4 est une vue en coupe d'une buse selon une quatrième forme de réalisation,
- la figure 4a est une vue en perspective d'une buse selon la figure 4,
- la figure 5 est une vue en plan en écorché d'une buse selon une autre forme de réalisation,
- la figure 5a est une vue en perspective de la buse selon la figure 5,
- la figure 6 est une vue en coupe longitudinale d'une buse selon une autre forme de réalisation,
- la figure 6a est une vue en perspective d'une buse selon la figure 6,
- la figure 7 est une vue en coupe longitudinale d'une buse selon une autre forme de réalisation,
- la figure 7a est une vue en perspective d'une buse selon la figure 7,
- la figure 8 est une vue en coupe longitudinale d'un appareil comportant une buse selon l'invention,
- la figure 8a est une vue en perspective d'un appareil selon la figure 8,
- la figure 9 est une vue partielle, en coupe en perspective d'un appareil doté de plusieurs buses de nébulisation,
- la figures 10 est une vue en coupe d'un appareil selon une autre forme de réalisation, installé sur un support,
- la figure 10a est une vue en perspective de l'appareil selon la figure 10,
- la figure 11 est une vue en coupe d'un appareil selon une autre forme de réalisation,
- la figure 11a est une vue en perspective de l'appareil selon la figure 11.

### Meilleure manière de réaliser l'invention

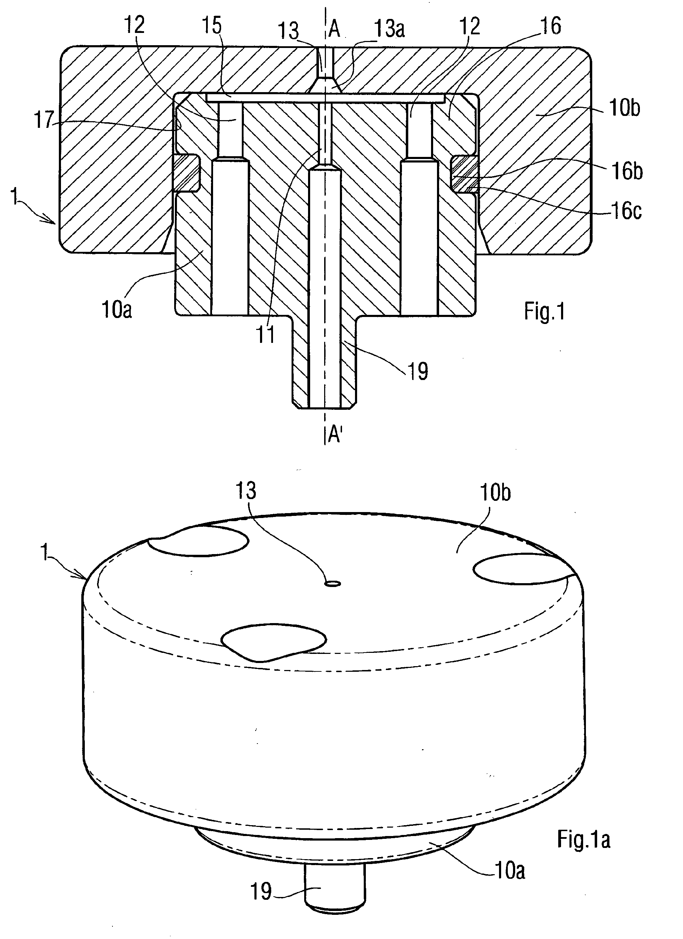

Telle que représentée sur les figures 1 à 7, la buse selon l'invention est formée d'un corps de buse rigide, de préférence cylindrique, dans lequel sont formés un premier perçage 11, axial au corps, d'amenée d'un gaz propulseur sous pression, ce premier perçage présentant un orifice d'entrée et un orifice de sortie, plusieurs seconds perçages 12 d'amenée d'un liquide à fractionner, chaque second perçage présentant un orifice d'entrée et un orifice de sortie et une zone 13 de fractionnement du liquide, axialement alignée avec le premier perçage selon un axe géométrique AA', préférentiellement rectiligne, ladite chambre de fractionnement étant en relation de communication avec les premier et seconds perçages.

Conformément à l'invention, la buse 1 est dotée d'une chambre de dépression 15 formée entre le premier perçage 11 et la zone de fractionnement 13, cette chambre 15 étant traversée par le flux de gaz propulseur et étant alimentée en liquide par les seconds perçages 12, les premier 11 et seconds perçages 12 débouchant dans ladite chambre par leur orifice de sortie. Comme on peut le voir sur les différentes figures, la chambre de dépression 15 comprend une face inférieure dans laquelle est formé l'orifice de sortie du perçage 11 d'amenée d'air, une face supérieure opposée à la précédente dans laquelle débouche la chambre de fractionnement 13 et une ou des faces latérales se développant entre les faces inférieure et supérieure.

Conformément à l'invention, les orifices de sortie des seconds perçages 12 sont disposés symétriquement par rapport à l'axe géométrique AA' et ce dit axe AA' est central à ladite chambre de dépression 15. Cette disposition assure une alimentation régulière et équilibrée de la chambre à dépression et de la zone de fractionnement.

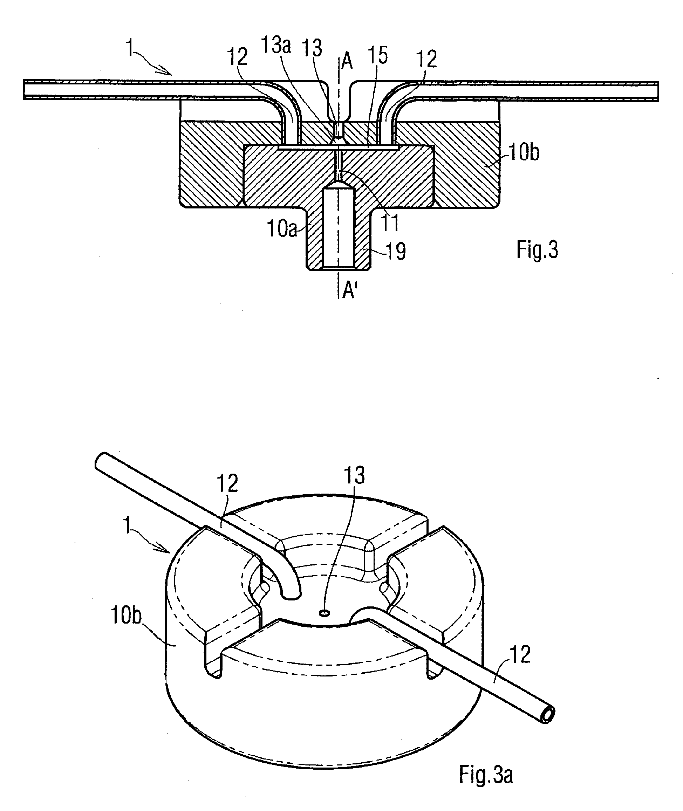

Selon une première forme de réalisation, telle que montrée sur les figures 1 à 5 et 7, les seconds perçages 12 sont latéraux et parallèles au premier perçage 11 et sont répartis de manière régulière autour de ce dernier. Dans cette forme de réalisation, les orifices de sortie des perçages d'amenée de liquide 12 sont formés dans la face inférieure de la chambre de dépression (Figs 1, 2, 4 5 et 7) ou sur la face supérieure plane de ladite chambre (Fig 3).

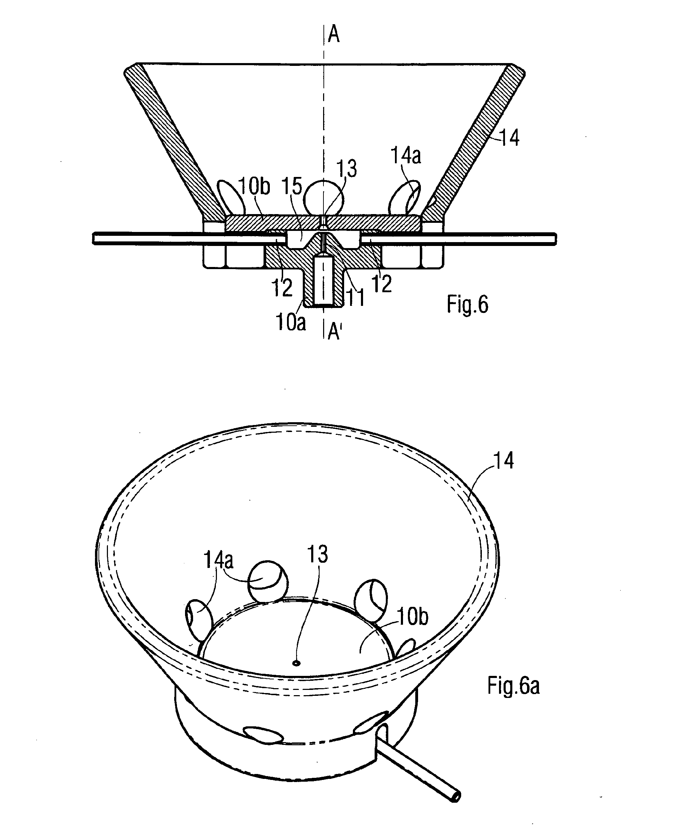

Selon une autre forme de réalisation comme on peut le voir en figure 6, les orifices de sortie des conduites d'amenée de liquide sont formés dans la ou les faces latérales de la chambre de dépression 15. Dans cette forme de réalisation la face inférieure de la chambre de dépression comportera un creux annulaire formé autour d'un bossage axial recevant le perçage 11 d'amenée de gaz porteur, l'orifice de sortie de chaque perçage 12 d'amenée de liquide étant formé dans la face latérale correspondante du creux annulaire.

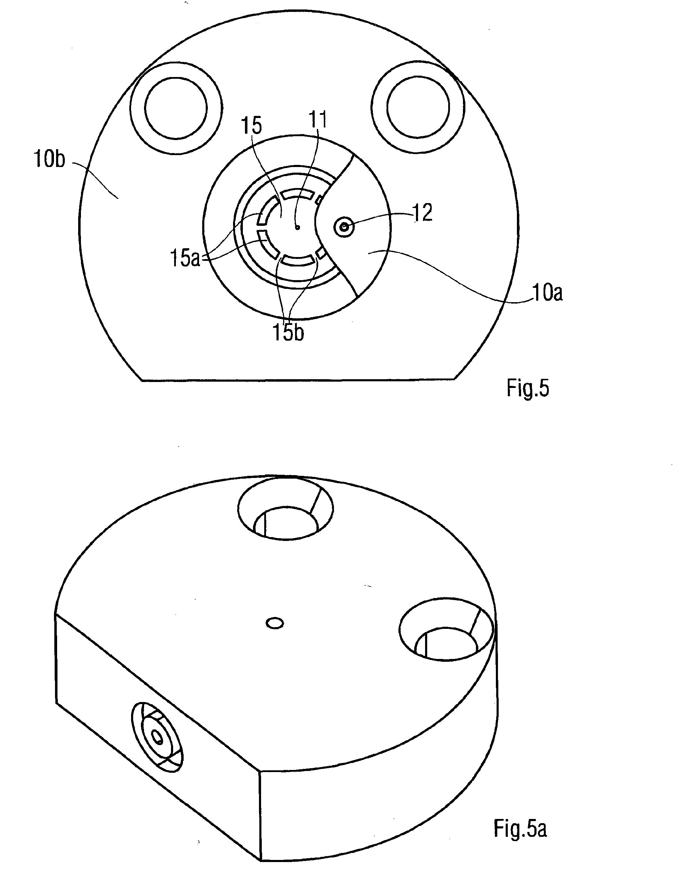

La chambre de dépression 15 comme on peut le voir plus particulièrement en figure 5 pourra être équipée d'une cloison annulaire 15a, centrée par rapport à l'axe AA', occupant toute sa hauteur et la divisant en un volume central traversé par le flux de gaz porteur et un volume annulaire, périphérique dans lequel débouchent les perçages 12 d'amenée de liquide. Cette paroi annulaire est pourvue de perçage radiaux 15b assurant la communication entre le volume annulaire et le volume central. Une telle disposition permet de créer une perte de charge contrôlée et convient pour une application de la buse en basse pression. De plus elle permet un meilleur équilibrage de l'arrivée de liquide. En outre pour une buse en matière synthétique, la paroi annulaire constitue un appui fèrme évitant toute déformation de la partie supérieure.

Avantageusement la chambre de dépression 15 est de forme cylindrique, mais en variante, cette chambre pourra être parallélépipédique.

Le volume de la chambre de dépression 15 est de préférence ajustable par variation de sa hauteur. L'ajustement du volume de la chambre outre le fait d'ajuster la quantité de liquide qu'elle peut admettre permet d'ajuster le degré de dépression régnant dans cette dernière, cette dépression étant créée par l'écoulement du gaz propulseur au travers de son volume interne entre l'ajutage du premier perçage 11 et la zone de fractionnement 13. Pour un débit de gaz propulseur égal, la dépression sera d'autant plus importante que la hauteur de la chambre 15 et donc son volume seront faibles. On conçoit également que par l'ajustement de la hauteur de la chambre est ajusté le débit du jet produit.

À titre d'exemple purement indicatif, pour un diamètre de chambre de 8 mm, la plage de réglage de la hauteur sera de 0,1 mm à 5 mm en fonction des applications et de l'intégration de la buse dans un ensemble mécanique ou autre élément.

La maîtrise de la valeur de la dépression est importante car elle permet dans le cadre de l'utilisation de la buse en atomisation d'aspirer un liquide sur une distance de 1 à 10 mètres avec une pression de 0,5 à 3 bars pour une consommation moyenne de 1 à 500 ml/heures en fonction du paramétrage de la chambre de dépression et du nombre de perçages d'amenée de liquide. Pour la nébulisation l'objectif est d'utiliser un micro compresseur basse tension (12Vcc) dans le but d'agir sur la tension ou par un PMW (variation du courant) pour augmenter ou diminuer le débit de l'aérosol.

Il est donc important d'avoir une dépression performante pour aspirer un liquide avec une pression la plus basse possible:
- hauteur moyenne d'aspiration de 20 à 500 mm
- pression mini de 15 à 30 mbars pour un débit d'air minimal de 11/mn
- consommation moyenne de 0,2 ml/h.
- débit compresseur 11/mn

La valeur maximum est de 12 Vcc pour une pression de 400 à 600 mbars et une consommation moyenne de liquide de 1,2 ml/h.

Ces valeurs sont données pour deux arrivés de liquide et un volume de chambre de l'ordre de 11 mm³

Le diamètre et la hauteur de perçage influent sur la dépression dans la chambre et sur le débit du micro compresseur au moment de l'amorçage.

Les tableaux ci-dessous montrent la variation d'amorçage de l'aspiration de liquide en fonction du diamètre et de la hauteur du perçage avec l'utilisation d'un micro compresseur.

| **Nébulisation Perçage 0,6mm / Hauteur 1mm** | **L/mn Débit compresseur** | **Pression en mbar** | **Tension Vcc** |
|---|---|---|---|
| Pression max | 2 | 570 | 12 |
| Pression mini | 1 | 60 | 4,5 |

| **Nébulisation Perçage 0,7mm / Hauteur 1mm** | **L/mn Débit compresseur** | **Pression en mbar** | **Tension Vcc** |
|---|---|---|---|
| Pression max | 2 | 560 | 12 |
| Pression mini | 1 | 50 | 4 |

| **Nébulisation Perçage 0,8mm / Hauteur 1mm** | **L/mn Débit compresseur** | **Pression en mbar** | **Tension Vcc** |
|---|---|---|---|
| Pression max | 2 | 560 | 12 |
| Pression mini | 1,2 | 50 | 4 |

| **Nébulisation Perçage 0,9mm / Hauteur 1mm** | **L/mn Débit compresseur** | **Pression en mbar** | **Tension Vcc** |
|---|---|---|---|
| Pression max | 2 | 560 | 12 |
| Pression mini | 1 | 50 | 4,2 |

| **Nébulisation Perçage 0,6mm / Hauteur 2mm** | **L/mn Débit compresseur** | **Pression en mbar** | **Tension Vcc** |
|---|---|---|---|
| Pression max | 2 | 550 | 12 |
| Pression mini | 1 | 60 | 4,5 |

| **Nébulisation Perçage 0,7mm / Hauteur 2mm** | **L/mn Débit compresseur** | **Pression en mbar** | **Tension Vcc** |
|---|---|---|---|
| Pression max | 2 | 580 | 12 |
| Pression mini | 1 | 30 | 3,8 |

| **Nébulisation Perçage 0,8mm / Hauteur 2mm** | **L/mn Débit compresseur** | **Pression en mbar** | **Tension Vcc** |
|---|---|---|---|
| Pression max | 2 | 580 | 12 |
| Pression mini | 1 | 20 | 3,5 |

| **Nébulisation Perçage 0,6mm / Hauteur 2mm** | **L/mn Débit compresseur** | **Pression en mbar** | **Tension Vcc** |
|---|---|---|---|
| Pression max | 2 | 560 | 12 |
| Pression mini | 1 | 30 | 4 |

Avantageusement, le corps de buse est formé de deux parties 10a, 10b, jointes de manière étanche l'une à l'autre, la chambre de dépression 15 étant formée entre et par ces deux parties de corps. Cette disposition est particulièrement avantageuse puisque la hauteur de la chambre 15 peut être facilement ajustée par éloignement ou rapprochement des deux parties l'une de l'autre. Ces deux parties de corps seront jointes l'une à l'autre par tous moyens connus. Elles pourront être jointes par des vis, par collage, sertissage, soudure etc.

Ces deux parties de corps pourront être axialement déplaçables l'une par rapport à l'autre. La hauteur de la chambre mesurée dans le sens de l'axe AA' est limitée à 5 mm pour une pression de 6 bars.

Dans la forme de réalisation objet des figures 1, 2, 4 et 7, les perçages 11, 12 et la chambre 15 sont formés dans la partie inférieure 10a tandis que la zone de fractionnement 13 est formée dans la partie supérieure 10b.

Dans la forme de réalisation objet de la figure 3 les perçages 12 d'amenée de liquide sont formés dans la partie supérieure 10b.

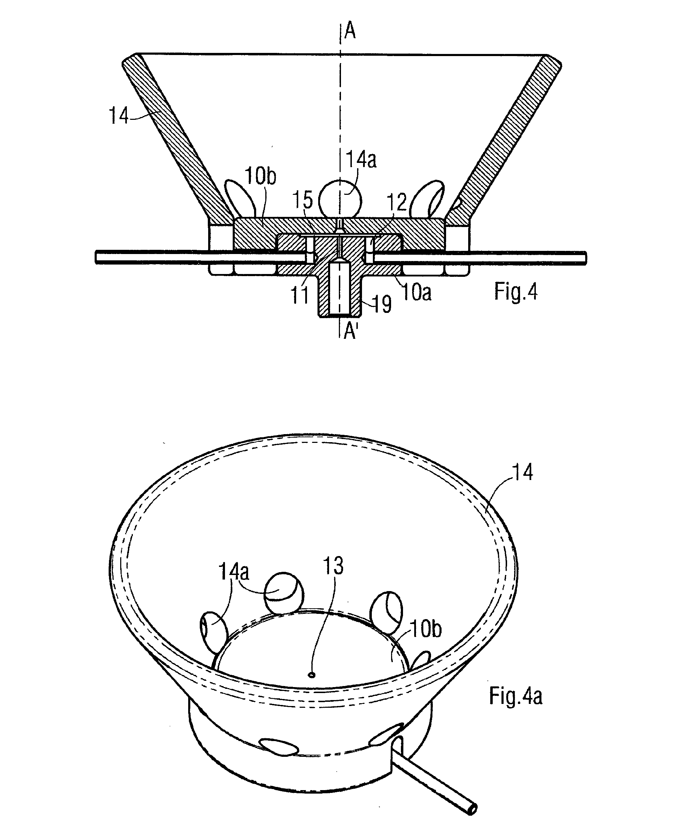

Dans la forme de réalisation de la figure 4 et de la figure 6, les perçages d'amenée de liquide 12 sont formés également dans la partie 10a et dans la partie 10b.

Dans la forme de réalisation de la figure 5, les perçages 12 d'amenée de liquide sont formées dans la partie 10a tandis que le perçage d'amenée d'air 11 est formée dans la partie 10b. Dans cette partie 10b est formée la chambre de dépression 15. La zone de fractionnement est formée dans la partie 10a.

Comme on peut le voir, en figures 1 à 6, la partie inférieure 10a de chaque buse objet de ces figures, présente une forme mâle d'embout cylindrique 16 axial, terminé par une surface plane perpendiculaire à l'axe du corps 10, depuis laquelle surface est creusée la chambre 15. La partie supérieure 10b présente une cavité cylindrique 17 de même diamètre que la forme d'embout cylindrique 16. Cette cavité engagée en ajustement glissant autour de la forme d'embout cylindrique 16. La cavité 17 présente un fond plat disposé en regard de la surface terminale de la forme d'embout cylindrique.

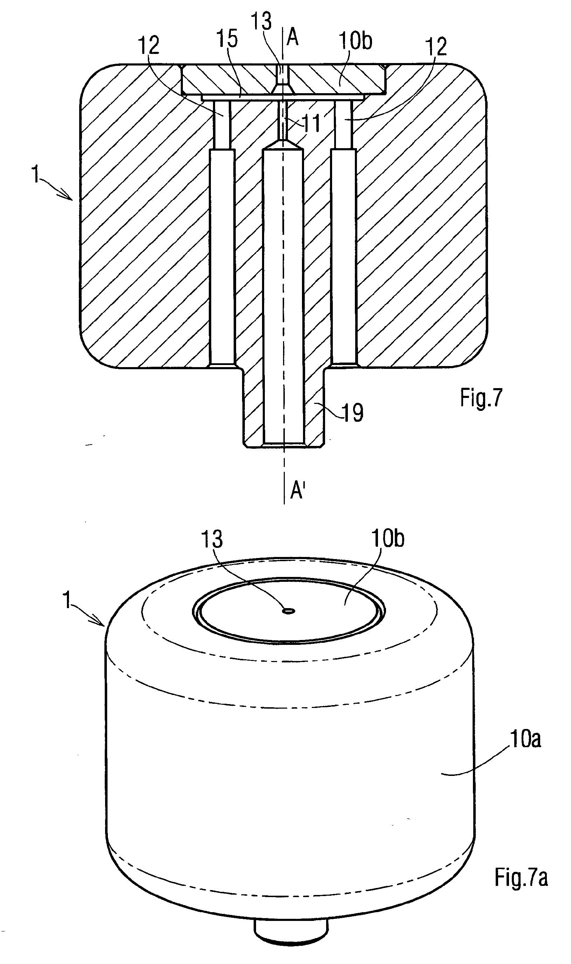

Dans la forme de réalisation de la buse objet de la figure 7, la partie supérieure supérieur 10b, sous forme de disque cylindrique, s'engage dans un logement cylindrique pratiqué dans la partie inférieure 10a.

La hauteur de la chambre 15 peut être déterminée par des cales sous forme d'anneaux, d'épaisseurs appropriées disposées entre les deux surfaces terminale de la forme d'embout 16 et de la cavité 17. Après mise en place des cales, les deux parties 10a, 10b de corps seront immobilisées l'une par rapport à l'autre, par exemple par des vis engagées d'une part dans des perçages traversants pratiqués dans la partie 10b de corps et dans des taraudages pratiqués dans la partie 10a de corps.

La hauteur de la chambre peut être déterminée par construction pour éviter l'usage de cales.

L'embout cylindrique 16 est doté d'une gorge 16b prévue pour recevoir un joint torique d'étanchéité 16c. Cette disposition en assurant l'étanchéité entre l'embout 16 et la cavité 17, s'oppose à toute fuite de liquide et de gaz propulseur.

La partie inférieure 10a de corps de buse est avantageusement équipée d'un embout axial 19 en saillie sur la face inférieure de ladite partie, et traversé par le premier perçage. Cet embout permet le raccordement du premier perçage à un conduit de distribution de gaz propulseur.

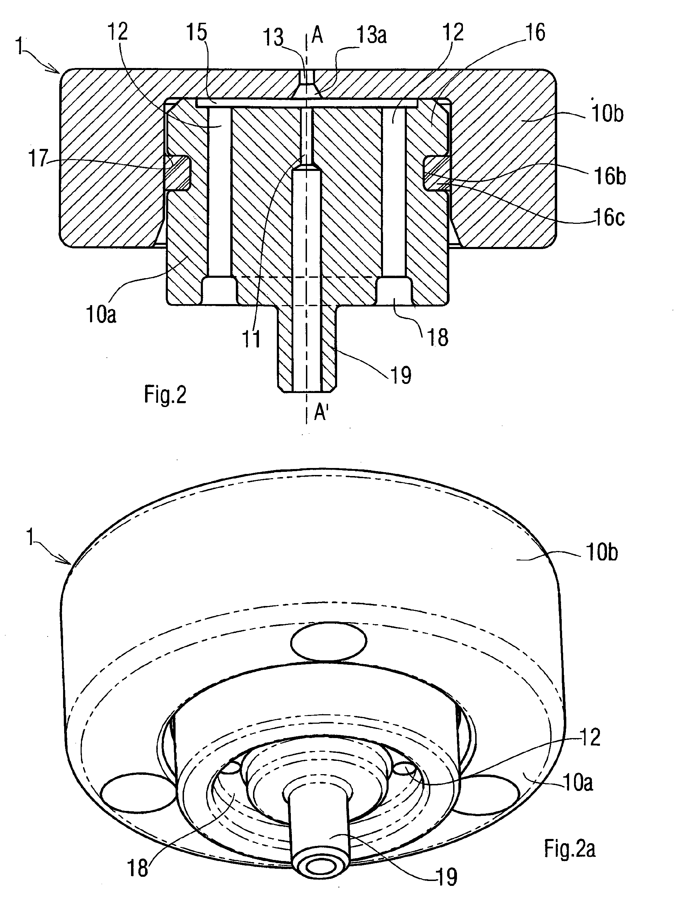

Selon une forme préférée de réalisation telle que montrée en figures 2 et 2a, le corps de buse 10 présente une gorge continue de distribution 18, s'étendant en cercle, dans laquelle débouchent les différents seconds perçages 12, ladite gorge étant destinée à être alimentée en liquide par une canule d'alimentation non représentée sur cette figure. Cette disposition permet de simplifier l'alimentation en liquide de la buse de nébulisation.

Comme on peut le voir, cette gorge 18 est pratiquée dans la partie inférieure 10a de corps et est creusée dans cette partie depuis la face inférieure plane que présente cette dernière. Cette face plane au moins au niveau de la gorge est destinée à être recouverte d'une cloison pour assurer l'étanchéité de la gorge de distribution 18. Cette cloison sera traversée de part en part par la canule d'alimentation sus évoquée.

De préférence, la zone de fractionnement 13 est formée par un perçage débouchant dans la chambre de dépression 15 et ce dans l'axe du premier perçage 11.

Le diamètre de la zone de fractionnement 13 est plus important que le diamètre du premier perçage de façon que l'accroissement de débit dû au liquide aspiré, puisse être facilement absorbé. De surcroît, le perçage constituant la zone de fractionnement 13 pourra présenter au niveau de son débouché dans la chambre de dépression et en regard du premier perçage, un évasement conique 13a. L'angle au sommet de cet évasement est compris entre 20 et 60 degrés. Cette disposition d'évasement favorise la mise en dépression de la chambre de dépression 15 et augmente l'efficacité du fractionnement du liquide.

Il y a lieu de noter que pour un fonctionnement de la buse sous haute pression, le diamètre de la zone de fractionnement sera suffisamment important pour éviter cette disposition d'évasement conique.

À la buse telle que décrite, peut être associé comme on peut le voir en figures 4 et 6 un pavillon de diffusion 14, en relation de communication avec la sortie de la zone 13 de fractionnement.

De préférence, le pavillon de diffusion 14 présente au moins deux prises d'air 14a radiales, débouchant dans son volume interne, lesdites prises d'air étant régulièrement réparties autour de son axe de symétrie.

Par effet Venturi une dépression est créée dans chaque prise d'air et de l'air est aspiré dans le pavillon de diffusion 14. Cette disposition augmente de manière particulièrement significative en sortie de buse tant le débit que la vitesse du jet. Comme on peut le voir ces prises d'air 14a sont formées à écartement de la zone de fractionnement et de préférence à proximité de la petite base du pavillon de diffusion.

Avantageusement, le pavillon de diffusion, présente un embout de fixation par emmanchement à la partie supérieure du corps de buse.

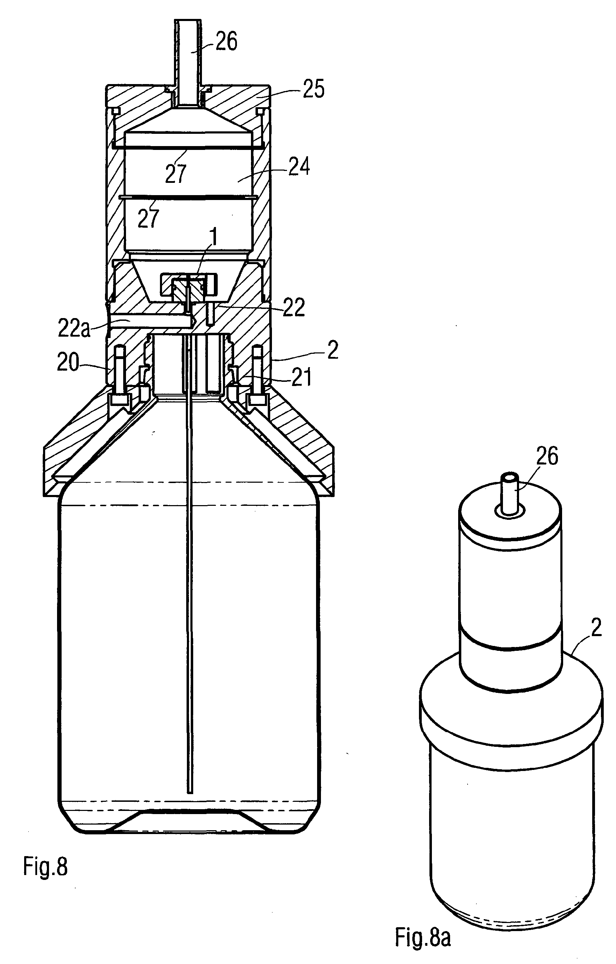

En figure 8 est représenté en coupe longitudinale un appareil 2 équipé d'une buse 1 conforme à l'invention.

Cet appareil comprend un corps creux 20 pourvu d'un embout 21 d'emmanchement au col d'un réservoir de liquide, d'une embase transversale 22 disposée au-dessus de l'embout d'emmanchement 21 dans laquelle est formé au moins un logement 23 apte à recevoir en emboîtement de forme, une buse de nébulisation 1 orientée dans le corps 20 de manière à produire un jet de particules de liquide orienté dans l'axe de l'appareil vers la partie supérieure de ce dernier.

L'embase transversale 22 présente un conduit 22a d'amené de gaz propulseur sous pression avec lequel vient en correspondance, le premier perçage 11 de la buse 1.

Dans la forme préférée de réalisation, le conduit de gaz propulseur est formé par un perçage borgne pratiqué dans l'embase de manière radiale. Selon l'axe longitudinal du corps, l'embase est dotée d'un second perçage borgne débouchant radialement dans le premier perçage borgne. Ce second perçage borgne constitue le logement 23 et reçoit l'embout de raccordement 19.

L'embase sera dotée de plus d'au moins un perçage traversant de passage d'une canule d'alimentation de la buse en liquide.

Le corps 20 de cet appareil comporte au-dessus de l'embase transversale et de la buse 1 au moins une chambre de détente 24 obturée en partie supérieure par une paroi amovible d'obturation 25 possédant une tubulure de sortie 26 de la nébulisation. Le jet produit par la buse s'introduit dans cette chambre de détente.

La chambre de détente formée dans le corps, est divisée transversalement par au moins une membrane 27, micro perforée, apte à ne laisser passer que les particules de taille inférieure à un calibre prédéterminé par exemple 3 µm. Cette chambre pourra être équipée ce deux membranes distantes l'une de l'autre et traversées successivement par la nébulisation.

De préférence de façon que les particules arrêtées par la membrane puissent s'évacuer d'elles mêmes vers le réservoir, l'embase, de manière latérale à la buse, présente des perçages traversants débouchant dans le volume de l'embout d'emmanchement 21.

Dans la forme de réalisation objet de la figure 8, l'appareil 2 ne comporte qu'une seule chambre de détente 24 mais en variante il pourra comporter deux chambres de détente 24 axialement alignées, chacune de ces chambres pouvant recevoir une ou plusieurs membranes 27 en fonction de l'application envisagée.

Cette configuration est principalement destinée à des applications pour lesquelles la pression en sortie de zone de nébulisation est comprise entre 0,5 et 3 bars.

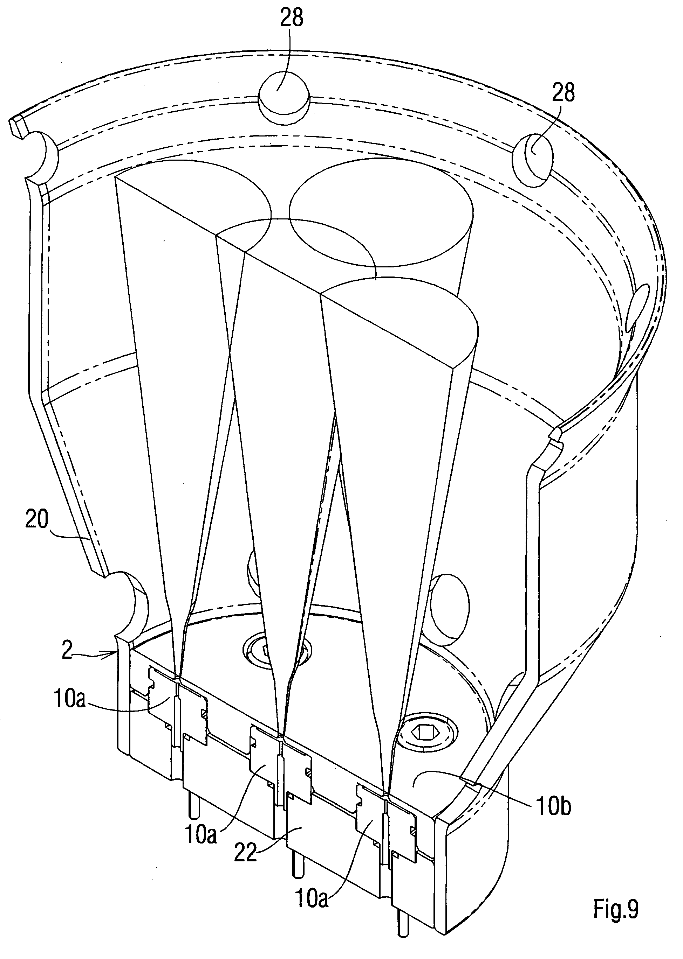

En figure 9 est représenté en coupe longitudinale un appareil comportant plusieurs buses 1 selon la seconde forme de réalisation. Ces buses 1 sont montées sur l'embase 22 de façon que les jets coniques qu'elles produisent, puissent s'entrecroiser. Selon cette forme de réalisation, le corps 20 de l'appareil forme au-dessus de l'embase une chambre ouverte en partie supérieure. Comme on peut le voir, le corps de l'appareil à proximité de l'extrémité supérieure de la chambre ouverte présente des orifice radiaux traversants 28, régulièrement répartis, constituant des prises d'air. En partie inférieure, immédiatement au dessus de l'embase 22, le corps 20 pourra également présenter d'autres orifices radiaux. Ainsi par effet Venturi de l'air est aspiré dans cette chambre ouverte afin d'augmenter la vitesse et le débit des jets produits.

On remarque sur cette figure que la partie 10b du corps de chaque buse est formée par une paroi commune fixée de manière amovible à l'embase 22.

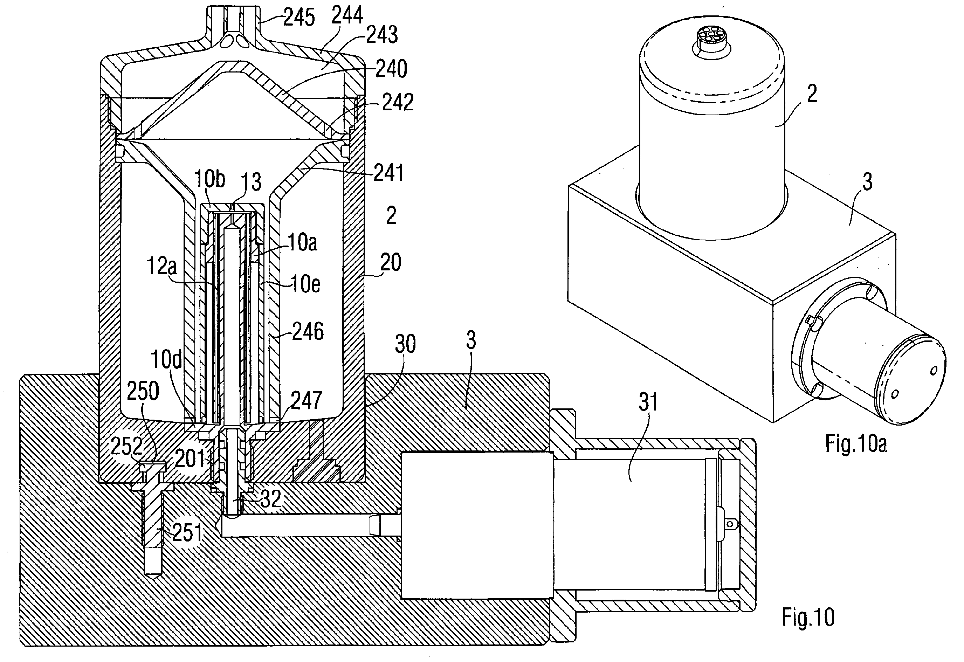

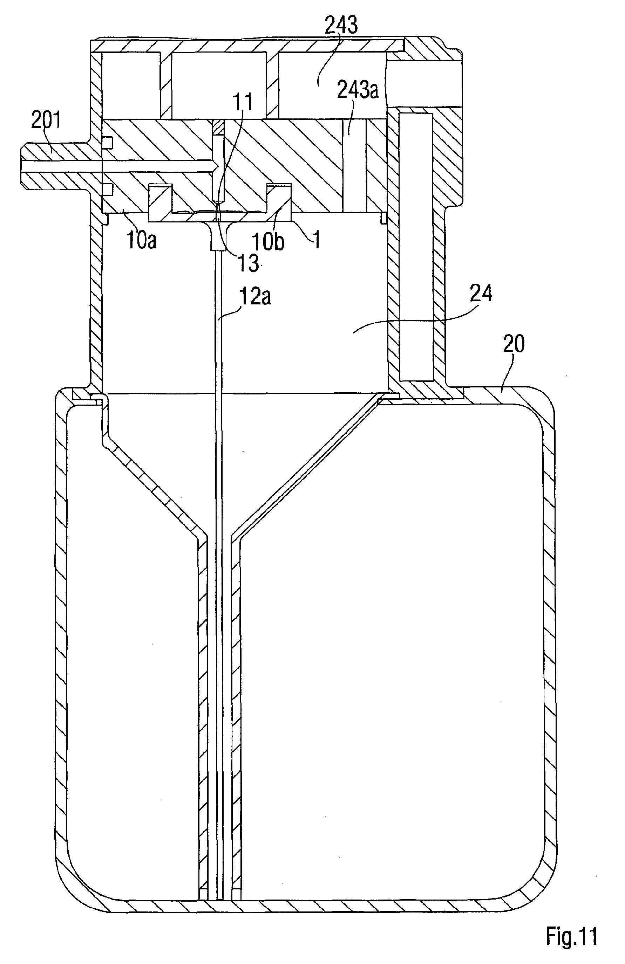

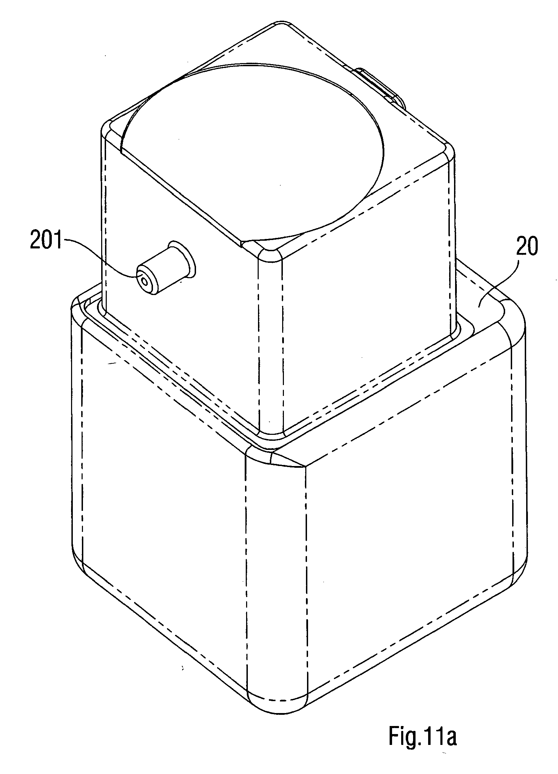

En figures 10 et 11 est représenté en coupe un appareil de nébulisation à usage unique.

On peut voir sur ces figures que le corps 20 de l'appareil constitue réservoir de liquide et que ce corps est doté d'un embout de raccordement 201 à une source d'air sous pression.

En figure 10 on remarque que l'embout de raccordement 201 est axial au corps cylindrique de l'appareil et occupe un position inférieure. Cet appareil est prévu pour être engagé dans un logement 30 formé dans support 3 recevant un compresseur d'air 31 constituant la source sous pression. On observe sur cette figure que l'embout 201 est du type femelle et reçoit en emboîtement de forme un embout mâle 32 raccordé à la source d'air sous pression. On remarque que cet embout mâle forme saillie sur le fond du logement 30.

La chambre de détente 24 est formée de deux cônes inversés l'un par rapport à l'autre 240, 241 joints l'un à l'autre par leur grande base, le cône inférieur 241 formant entonnoir de récupération des écoulements résiduels. Le cône supérieur 240 est doté de perçages traversants 241 d'échappement de la nébulisation vers une chambre supérieure 243 formée entre ledit cône supérieur 240 et une coiffe supérieure 244 que comporte l'appareil. Les perçages traversants 241 sont formés à proximité de la grande base du cône supérieur.

Cette coiffe supérieure 244 est dotée d'un embout de sortie de la nébulisation 245 et se fixe de manière étanche au corps de l'appareil.

Avantageusement, le cône inférieur 241 est prolongé vers le fond du réservoir par une forme tubulaire 246 prenant appui sur une embase 10d fixée au corps longiforme de la partie inférieure 10a de buse. Entre cette forme tubulaire 246 et le corps longiforme 10a est ménagée une chambre annulaire en relation de communication avec le réservoir au travers de perçages traversants radiaux 247 ménagés dans cette forme tubulaire, en partie inférieure. On peut observer également que cette chambre annulaire est en relation de communication avec la chambre de détente 24.

On peut également observer que l'embase 10d présente une gorge radiale d'alimentation d'une canule d'aspiration de liquide 12a que comporte la buse, cette canule d'aspiration étant en relation de communication avec le perçage 12 correspondant. On peut observer que cette canule d'aspiration 12a est montée dans un logement longiforme formé dans la partie 10a de corps de buse. Autour de la partie 10a sera disposé un fourreau tubulaire 10e prenant appui sur l'embase 10d. Ce fourreau tubulaire est doté en extrémité inférieure de perçages traversant de passage du liquide à nébuliser.

On peut voir sur cette figure que l'appareil comporte un moyen d'indexage et de blocage dans constitué par une rainure 250 en arc de circonférence de cercle de section droite en Té pratiquée dans la paroi de fond du corps d'appareil dans laquelle est engagée un pion 251 avec tête 252 annulairement saillante prévue pour être engagée dans le segment horizontal du Té que forme la gorge.

En figure 11 est représentée une autre forme de réalisation d'un appareil de nébulisation à usage unique, comportant un réservoir de liquide formé dans le corps 20 d'appareil. Cet appareil comporte par exemple une buse 1 selon la figure 5. On peut voir sur cette figure que la pulvérisation formée en sortie de buse est orientée vers une chambre de détente inférieure 24 formée au dessus du niveau de liquide contenu dans le réservoir. La paroi inférieure de cette chambre 24 est agencée en entonnoir de récupération des écoulements résiduels. Comme on peut le voir cet entonnoir possède une canule verticale qui s'enfonce dans le réservoir. L'extrémité inférieure de cette canule verticale prend appui sur le fond du réservoir et présente des perçages radiaux traversants. Des canules d'aspiration 12a de liquide, en relation de communication avec les perçages 12 de la buse sont engagées dans la canule de l'entonnoir et puisent en partie inférieure de ce dernier le liquide contenu dans le corps 20 ce dernier formant réservoir. La chambre de détente 24 est en relation de communication avec une chambre supérieure 243 par un perçage traversant 243a pratiqué dans le corps de buse, cette chambre comportant un orifice de sortie de la nébulisation. On remarque sur cette figure que l'entrée d'air 201 est radiale au corps de l'appareil.

Il va de soi que la présente invention peut recevoir tous aménagements et variantes du domaine des équivalents techniques sans pour autant sortir du présent brevet.

## Revendications

1. Buse (1) de pulvérisation formée d'un corps de buse comportant un premier perçage (11) d'amenée de gaz sous pression, présentant un orifice d'entrée et un orifice de sortie, plusieurs seconds perçages (12) d'amenée d'un liquide à fractionner présentant un orifice d'entrée et un orifice de sortie, une zone de fractionnement(13) du liquide, axialement alignée avec le premier perçage (11) selon un axe géométrique AA', ladite zone de fractionnement (13) étant en relation de communication avec les premier (11) et seconds (12) perçages, lesquels sont en relation de communication avec le premier perçage (11) et avec la zone de fractionnement (13) au travers d'une même chambre de dépression (15) formée entre ledit premier perçage (11) et la zone de fractionnement (13), ladite chambre de dépression (15) étant traversée selon l'axe AA' par le flux de gaz propulseur et le diamètre de la zone de fractionnement (13) étant plus important que le diamètre du premier perçage (11) au niveau de son débouché dans la chambre de dépression (15), l'ajutage du premier perçage (11) au niveau du débouché de ce dernier dans la chambre de dépression (15) étant axial à ladite chambre, les ajutages des seconds perçages (12) au niveau des débouchés de ces derniers dans ladite chambre (15) étant régulièrement répartis autour de l'axe AA', la zone de fractionnement (13) étant formée par un perçage débouchant dans la chambre de dépression (15) et ce dans l'axe du premier perçage (11),
**caractérisée en ce que** :
- le volume de la chambre de dépression (15) est ajustable.

2. Buse (1) de pulvérisation selon la revendication précédente, **caractérisée en ce que** le perçage constituant la zone de fractionnement (13) présente au niveau de son débouché dans la chambre de dépression et en regard du premier perçage, un évasement conique.

3. Buse (1) de pulvérisation selon l'une quelconque des revendications précédentes, **caractérisée en ce que** le corps de buse est formé de deux parties (10a, 10b) jointes de manière étanche l'une à l'autre, la chambre de dépression (15) étant formée entre et par ces dernières.

4. Buse (1) de pulvérisation selon l'une quelconque des revendications précédentes, **caractérisée en ce que** le corps de buse reçoit un pavillon (14) de diffusion de l'aérosol formé par le gaz propulseur et par le liquide fractionné, ledit pavillon étant en relation de communication avec la zone de pulvérisation (13) et que le pavillon de diffusion (14) présente au moins deux prises d'air (14a) débouchant dans son volume interne, les dites prises d'air(14a) étant régulièrement réparties autour de l'axe de symétrie dudit pavillon (14).

5. Buse (1) de pulvérisation selon l'une quelconque des revendications précédentes, **caractérisée en ce que** la chambre à dépression est équipée d'une cloison annulaire (15a), centrée par rapport à l'axe AA', occupant toute sa hauteur et la divisant en un volume central traversé par le flux de gaz porteur et un volume annulaire, périphérique dans lequel débouchent les perçages (12) d'amenée de liquide ladite paroi annulaire étant pourvue de perçages radiaux (15b) assurant la communication entre le volume annulaire et le volume central.

6. Appareil de nébulisation, **caractérisé en ce qu'**il comporte au moins une buse (1) selon l'une quelconque des revendications précédentes.

7. Appareil de nébulisation, selon la revendication précédente, **caractérisé en ce qu'**il présente au-dessus ou au dessous de la buse (1), au moins une chambre de détente (24).

8. Appareil de nébulisation, selon la revendication 6 ou la revendication 7, **caractérisé en ce qu'**il comprend un corps creux (20) d'appareil pourvu d'un embout d'emmanchement (21) sur le col d'un réservoir de liquide, d'une embase transversale (22) disposée au-dessus de l'embout d'emmanchement dans laquelle est formé au moins un logement (23) apte à recevoir en emboîtement de forme une buse de nébulisation (1).

9. Appareil de nébulisation selon l'une quelconque des revendications 6 à 8, **caractérisé en ce que** l'embase (22) présente un conduit (22a) d'amené de gaz propulseur sous pression avec lequel vient en correspondance le perçage (11) de la buse (1).

10. Appareil de nébulisation selon l'une quelconque des revendications 6 à 9, **caractérisé en ce que** la chambre de détente (24) reçoit au moins une membrane transversale (27), micro-perforée apte à ne laisser passer que les particules de liquides de tailles inférieures à un calibre prédéterminé.

11. Appareil de nébulisation selon l'une quelconque des revendications 6 à 11, **caractérisé en ce qu'**il présente plusieurs buses (1) montées sur l'embase (22) de façon que les jets coniques qu'elles produisent, s'entrecroisent.

12. Appareil de nébulisation à usage unique selon la revendication 6, **caractérisé en ce que** le corps (20) constitue réservoir de liquide.

13. Appareil selon la revendication précédente, **caractérisé en ce que** la chambre de détente (24) est formée de deux cônes inversés joints l'un à l'autre par leur grande base, le cône inférieur formant entonnoir de récupération des écoulements résiduels, et le cône supérieur étant doté de perçages traversants d'échappement de la nébulisation vers une chambre supérieure formée entre ledit cône supérieur et une coiffe supérieure, ladite coiffe supérieure étant dotée d'un embout de sortie de la nébulisation.

14. Appareil de nébulisation selon la revendication précédente, **caractérisé en ce que** le cône inférieur (241) est prolongé vers le fond du réservoir par une forme tubulaire (246) prenant appui sur une embase (10d) fixée au corps longiforme de la partie inférieure (10a) de buse et qu'entre cette forme tubulaire (246) et le corps longiforme (10a) est ménagée une chambre annulaire en relation de communication avec le réservoir au travers de perçages traversants radiaux (247) ménagés dans ladite forme tubulaire, en partie inférieure.

15. Appareil selon la revendication précédente, **caractérisé en ce que** l'embase (10d) présente au droit de chaque perçage radial (247) une gorge radiale d'alimentation d'une canule d'aspiration de liquide (12a) que comporte la buse (1), ladite canule d'aspiration (12a) étant montée dans un logement longiforme formé dans la partie (10a) de corps de buse et qu'autour de la partie (10a) est disposé un fourreau tubulaire (10e) prenant appui sur l'embase (10d), ledit fourreau tubulaire étant doté en extrémité inférieure de perçages traversant de passage du liquide à nébuliser.

## Patentansprüche

1. Sprühdüse (1), die von einem Düsenkörper gebildet wird, welcher eine erste Bohrung (11) zum Heranfahren eines unter Druck stehenden Gases aufweist, wobei diese mit einer Eintrittsöffnung und einer Austrittsöffnung versehen ist, sowie mehrere zweite Bohrungen (12) zum Heranführen einer zu zerstäubenden Flüssigkeit, wobei diese mit einer Eintrittsöffnung und einer Austrittsöffnung versehen sind, einen Bereich (13) zum Zerstäuben der Flüssigkeit, wobei der Zerstäubungsbereich (13) axial gemäß einer ersten geometrischen Achse AA' auf die erste Bohrung (11) ausgerichtet ist und mit der ersten (11) und mit den zweiten (12) Bohrungen in Verbindung steht, wobei letztere über eine Unterdruckkammer (15) mit der ersten Bohrung (11) und mit dem Zerstäubungsbereich (13) in Verbindung stehen, wobei die Unterdruckkammer (15) zwischen der ersten Bohrung (11) und dem Zerstäubungsbereich (13) gebildet ist und gemäß der Achse AA' vom Treibgasstrom durchquert wird und wobei der Durchmesser des Zerstäubungsbereichs (13) größer als der Durchmesser der ersten Bohrung (11) ist, welchen diese auf Höhe ihrer Einmündung in die Unterdruckkammer (15) aufweist, wobei die erste Bohrung (11) auf Höhe ihrer Einmündung in die Unterdruckkammer (15) ein Ansatzstück aufweist, das axial zu dieser Kammer ist, wobei die zweiten Bohrungen (12) auf Höhe ihrer Einmündungen in die Kammer (15) Ansatzstücke aufweisen, die gleichmäßig um die Achse AA' verteilt sind, wobei der Zerstäubungsbereich (13) durch eine Bohrung gebildet wird, welche in die Unterdruckkammer (15) einmündet, und zwar in der Achse der ersten Bohrung (11),
**dadurch gekennzeichnet, dass**:
- das Volumen der Unterdruckkammer (15) einstellbar ist.

2. Sprühdüse (1) nach dem vorhergehenden Anspruch, **dadurch gekennzeichnet, dass** die Bohrung, aus welcher der Zerstäubungsbereich (13) besteht, auf Höhe ihrer Einmündung in die Unterdruckkammer und der ersten Bohrung gegenüberliegend eine kegelförmige Erweiterung aufweist.

3. Sprühdüse (1) nach einem beliebigen der vorhergehenden Ansprüche, **dadurch gekennzeichnet, dass** der Düsenkörper aus zwei Abschnitten (10a, 1b) gebildet ist, die abdichtend miteinander verbunden sind, wobei die Unterdruckkammer (15) derart von den letzteren gebildet ist, dass sie sich zwischen diesen befindet.

4. Sprühdüse (1) nach einem beliebigen der vorhergehenden Ansprüche, **dadurch gekennzeichnet, dass** der Düsenkörper einen Trichter (14) aufnimmt, der zur Abgabe des Aerosols dient, welches sich aus dem Treibgas und der zerstäubten Flüssigkeit bildet, wobei der Trichter mit dem Zerstäubungsbereich (13) in Verbindung steht, und dass der Abgabetrichter (14) mindestens zwei Lufteinlässe (14a) aufweist, die in seinen Innenraum einmünden, wobei die Lufteinlässe (14a) gleichmäßig um die Symmetrieachse des Trichters (14) verteilt sind.

5. Sprühdüse (1) nach einem beliebigen der vorhergehenden Ansprüche, **dadurch gekennzeichnet, dass** die Unterdruckkammer mit einen ringförmigen Trennwand (15a) versehen ist, die unter Bezugnahme auf die Achse AA' mittig ausgerichtet ist, wobei sie die gesamte Höhe der Kammer einnimmt und diese derart unterteilt, dass ein Mittelraum entsteht, welcher von einem Trägergasstrom durchquert wird, und ein randständiger Ringraum, in welchen die Bohrungen (12) zum Heranfuhren der Flüssigkeit einmünden, wobei die ringförmige Wand mit radialen Bohrungen (15b) versehen ist, die eine Verbindung zwischen dem Ringraum und dem Mittelraum sicherstellen.

6. Sprühgerät, **dadurch gekennzeichnet, dass** es mindestens ein Düse (1) nach einem beliebigen der vorhergehenden Ansprüche aufweist.

7. Sprühgerät nach dem vorhergehenden Anspruch, **dadurch gekennzeichnet, dass** es oberhalb oder unterhalb der Düse (1) mindestens eine Entspannungskammer (24) aufweist.

8. Sprühgerät nach Anspruch 6 oder Anspruch 7, **dadurch gekennzeichnet, dass** es einem Gerätehohlkörper (20) umfasst, der mit einem Aufsteckstutzen (21) versehen ist, welche auf dem Hals eines Flüssigkeitsbehälters sitzt, sowie mit einer querverlaufenden Platte (22), welcher oberhalb des Aufsteckstutzens angeordnet ist und in welcher mindestens ein Aufnahmeraum (23) gebildet ist, der eine Sprühdüse (1) formschlüssig aufnehmen kann.

9. Sprühgerät nach einem beliebigen der Ansprüche 6 bis 8, **dadurch gekennzeichnet, dass** die Platte (22) eine Leitung (22a) aufweist, welche dem Heranführen von unter Druck stehendem Treibgas dient und mit welcher die Bohrung (11) der Düse (1) in Verbindung gebracht wird.

10. Sprühgerät nach einem beliebigen der Ansprüche 6 bis 9, **dadurch gekennzeichnet, dass** die Entspannungskammer (24) mindestens eine querverlaufende Membran (27) aufnimmt, die mikroperforiert ist und derart ausgebildet ist, dass sie nur Flüssigkeitspartikel durchlässt, deren Größe geringer als ein vorbestimmtes Durchgangsmaß ist.

11. Sprühgerät nach einem beliebigen der Ansprüche 6 bis 11, **dadurch gekennzeichnet, dass** es mehrere Düsen (1) aufweist, die derart auf der Platte (22) angebracht sind, dass sie kegelförmige Strahlen erzeugen, die sich kreuzen.

12. Sprühgerät zum Einmalgebrauch nach Anspruch 6, **dadurch gekennzeichnet, dass** der Körper (20) den Flüssigkeitsbehälter bildet.

13. Gerät nach dem vorhergehenden Anspruch, **dadurch gekennzeichnet, dass** die Entspannungskammer (24) aus zwei entgegengesetzten Kegeln gebildet ist, die mit ihrer großen Basis aneinandergefügt sind, wobei der untere Kegel einen Auffangtrichter für restliche Stoffströme bildet und der obere Kegel mit Durchgangsbohrungen versehen ist, durch welche der Sprühnebel in eine obere Kammer entweichen kann, die zwischen dem oberen Kegel und einer oberen Haube gebildet ist, wobei die obere Haube mit einem Stutzen zum Austritt des Sprühnebels versehen ist.

14. Sprühgerät nach dem vorhergehenden Anspruch, **dadurch gekennzeichnet, dass** der untere Kegel (241) sich in Richtung des Bodens des Behälters in einem röhrenförmigen Stück (246) fortsetzt, das auf einer Platte (10d) aufliegt, welche an dem länglichen Körper des unteren Abschnitts (10a) der Düse befestigt ist, und dass zwischen dem röhrenförmigen Stück (246) und dem länglichen Körper (10a) eine ringförmige Kammer ausgespart ist, die im unteren Abschnitt über radiale Durchgangsbohrungen (247), welche in dem röhrenförmigen Stück ausgespart sind, mit dem Behälter in Verbindung steht.

15. Gerät nach dem vorhergehenden Anspruch, **dadurch gekennzeichnet, dass** die Platte (10d) jeder der radialen Bohrungen (247) gegenüberliegend eine radiale Hohlkehle zur Versorgung einer Flüssigkeitsansaugkanüle (12a) aufweist, mit welcher die Düse (1) versehen ist, wobei die Ansaugkanüle (12a) in einem länglichen Aufnahmeraum angebracht ist, welcher innerhalb des Abschnitts (10a) des Düsenkörpers gebildet ist, und dass rundum den Abschnitt (10a) eine röhrenförmiger Hülse (10e) angeordnet ist, die auf der Platte (10d) aufliegt, wobei die röhrenförmige Hülse am unteren Ende mit Durchgangsbohrungen zum Durchlass der zu versprühenden Flüssigkeit versehen ist.

## Claims

1. Spray nozzle (1) formed of a nozzle body comprising a first bore (11) delivering pressurized gas, having an inlet port and an outlet port, several second bores (12) delivering a liquid to be fractionated, having an inlet port und an outlet port, a liquid fractionation area (13), axially aligned with the first bore (11) along a geometric axis AA', said fractionation area (13) being in communication with the first (11) and second (12) bores, which are in communication with the first bore (11) and the fractionation area (13) through a single vacuum chamber (15) formed between said first bore (11) und the fractionation area (13), said vacuum chamber (15) being crossed along the axis AA' by the flow of propellant gas and the diameter of the fractionation area (13) being larger than the diameter of the first bore (11) at the location where it emerges in the vacuum area (15), the orifice of the first bore (11) at the location where it emerges in the vacuum chamber (15) being axial to said chamber, the orifices of the second bores (12) at the location where they emerge in said chamber (15) being distributed regularly around the axis AA', the fractionation area (13) being formed by a bore emerging in vacuum chamber (15) in the axis of the first bore (11), **characterized in that**:
the volume of the vacuum chamber (15) is adjustable.

2. Spray nozzle (1) according to the preceding claim, **characterized in that** the bore forming the fractionation area (13) has, at the location where it emerges in the vacuum chamber und opposite the first bore, a conical flaring.

3. Spray nozzle (1) according to any one of the preceding claims, **characterized in that** the nozzle body is formed of two portions (10a, 10b) joined hermetically together, the vacuum chamber (15) being formed between and by these portions.

4. Spray nozzle (1) according to any one of the preceding claims, **characterized in that** the nozzle body receives a bellmouth (14) for diffusing the aerosol formed by the propellant gas and the fractionated liquid, said bellmouth being in communication with the spray area (13), and **in that** the diffusion bellmouth (14) has at least two air inlets (14a), emerging in its interior volume, said air inlets (14a) being distributed regularly around the axis of symmetry of said bellmouth (14).

5. Spray nozzle (1) according to any one of the preceding claims, **characterized in that** the vacuum chamber is equipped with an annular partition (15a), centered relative to the axis AA', occupying its entire height and dividing it into a central volume, crossed by the carrier gas flow, and a peripheral annular volume, into which the liquid delivery bores (12) emerge, said annular partition being provided with radial bores (15b) providing communication between the annular volume and the central volume.

6. Atomizing device, **characterized in that** it comprises at least one nozzle (1) according to any one of the preceding claims.

7. Atomizing device according to the preceding claim, **characterized in that** it has, above or below the nozzle (1), at least one expansion chamber (24).

8. Atomizing device according to claim 6 or claim 7, **characterized in that** it comprises a hollow device body (20) provided wit an end-fitting (21) for coupling onto the neck of a liquid reservoir, a transverse baseplate (22) positioned above the coupling end-fitting wherein is formed at least one housing (23) able to receive, as a complementary shape, an atomization nozzle (1).

9. Atomizing device according to any one of claims 6 to 8, **characterized in that** the baseplate (22) has a duct (22a) for delivering pressurized propellant gas with which the bore (11) of the nozzle (1) enters into correspondence.

10. Atomizing device according to any one of claims 6 to 9, **characterized in that** the expansion chamber (24) receives at least one transverse micro-perforated membrane (27), able to let only those liquid particles with sizes less than a predefined caliber pass through.

11. Atomizing device according to any one of claims 6 to 11, **characterized in that** it has several nozzles (1) mounted on the baseplate (22) such that the conical jets they produce cross each other.

12. Single-use atomizing device according to claim 6, **characterized in that** the body (20) constitutes a liquid reservoir.

13. Device according to the preceding claim, **characterized in that** the expansion chamber (24) is formed of two inverted cones joined together by their large base, the lower cone forming a funnel for collecting residual flows, and the upper cone being equipped with through holes for venting the atomization towards an upper chamber formed between said upper cone and an upper cover, said upper cover being equipped with an atomization outlet end-fitting.

14. Atomizing device according to the preceding claim, **characterized in that** the lower cone (241) is extended towards the bottom of the reservoir by a tubular form (246) resting on a baseplate (10d) fixed to the elongated body of the nozzle's lower portion (10a), and **in that**, between this tubular form (246) and the elongated body (10a), an annular chamber is formed, in communication with the reservoir through radial through holes (247) formed in said tubular form, in the lower portion.

15. Device according to the preceding claim, **characterized in that** the baseplate (10d) has, in line with each radial hole (247), a radial channel supplying a liquid aspiration tube (12a) that the nozzle (1) includes, said aspiration tube (12a) being mounted in an elongated housing formed in the nozzle body portion (10a), and **in that** around portion (10a) is positioned a tubular sleeve (10e) resting on the baseplate (10d), said tubular sleeve being equipped at the lower end with through holes for the passage of the liquid to be atomized.
